**Europäisches Patentamt**

**European Patent Office**

**Office européen des brevets**

(19)

(11) Veröffentlichungsnummer: **0 017 827**
**B2**

(12) **NEUE EUROPÄISCHE PATENTSCHRIFT**

(45) Veröffentlichungstag der neuen Patentschrift:
**07.12.88**

(51) Int. Cl.⁴: **C 07 C 51/41,** C 09 F 9/00,
C 11 D 9/00

(21) Anmeldenummer: **80101664.3**

(22) Anmeldetag: **27.03.80**

(54) **Verfahren zur Herstellung von Metallseifen.**

(30) Priorität: **04.04.79 DE 2913592**

(43) Veröffentlichungstag der Anmeldung:
**29.10.80 Patentblatt 80/22**

(45) Bekanntmachung des Hinweises auf die Patenterteilung:
**31.03.82 Patentblatt 82/13**

(45) Bekanntmachung des Hinweises auf die Entscheidung
über den Einspruch:
**07.12.88 Patentblatt 88/49**

(84) Benannte Vertragsstaaten:
**AT BE CH DE FR GB IT LU NL SE**

(56) Entgegenhaltungen:
**DE-A- 1 493 310**
**DE-A- 2 002 185**
**DE-A- 2 361 607**
**DE-A- 2 619 958**
**DE-B- 1 068 238**
**DE-B- 1 279 658**
**GB-A- 1 406 526**
**US-A- 2 890 232**
**US-A- 3 519 371**

**C.J.Boner: Manufacture and application of lubricating
greases, New York (1954)
S.B. Elliott: The alkaline-earth and heavy-metal soaps,
New York (1946)**

(73) Patentinhaber: **Chemische Werke München Otto
Bärlocher GmbH, Riesstrasse 16,
D-8000 München 50 (DE)**

(72) Erfinder: **Pietralia, Norbert, Weidenstrasse 33,
D-8034 Unterpfaffenhofen (DE)**
Erfinder: **Ausserbauer, Valentin, Zugspitzweg 29,
D-8015 Markt Schwaben (DE)**
Erfinder: **Rosenthal, Christian, Dr. Dipl.-Chem.,
Bäckergasse 15, D-8131 Berg (DE)**

(74) Vertreter: **Patentanwälte Dipl.-Ing. A. Grünecker,
Dr.-Ing. H. Kinkeldey, Dr.-ing. W. Stockmair,, Dr. rer. nat.
K. Schumann, Dipl.-Ing. P.H. Jakob, Dr. rer. nat. G.
Bezold Maximilianstrasse 58, D-8000 München 22 (DE)**

(56) Entgegenhaltungen: (Fortsetzung)
**Chemical Engineering Vol. 2, 2nd Ed. Pergamon Press
68 P. 713
J.M. Coulson, J.F. Richard: Chemical Engineering 2nd
Edition, Vol. 2 - Oxford 81968) p. 713
The Merck Index 8th Edition, Rahway (1968), p. 131**

## Beschreibung

Die Erfindung betrifft ein Verfahren zur Herstellung von Metallseifen oder Metallseifengemischen durch Umsetzen mindestens einer festen aliphatischen Carbonsäure mit 8 bis 22 Kohlenstoffatomen mit mindestens einem Metalloxid und/oder -hydroxid und/oder -carbonat aus der Gruppe Lithium, Natrium, Kalium, Magnesium, Calcium, Strontium, Barium, Zinn, Blei und Zink in mindestens einer zur Carbonsäure äquivalenten Menge bis zu einem 2%igen Überschuss in Gegenwart von 1 bis 5% Wasser, bezogen auf das Gesamtgewicht der Carbonsäure(n), bei erhöhter Temperatur, wobei man das Gemisch auf eine für das Anspringen der Umsetzung nötige Temperatur erhitzt, die exotherme Reaktion in einem geschlossenen Druckreaktor ablaufen lässt, anschliessend vom aufgebauten Überdruck entspannt und dann das im Reaktionsgemisch enthaltene Wasser entfernt.

Es ist bereits ein Verfahren bekannt, bei dem durch doppelte Umsetzung der Carbonsäure die Metallseife gewonnen wird. Hierbei erfolgt zuerst Umsetzung mit einer äquivalenten Menge Natronlauge oder Kalilauge zu der entsprechenden Alkaliseife. In einem weiteren Schritt wird diese Alkaliseife mit dem jeweiligen Metallsalz versetzt und die sich bildende, unlösliche Metallseife ausgefällt. Diese Umsetzung erfolgt bei Temperaturen zwischen 60 und 70°C in grossem Überschuss an Wasser, und zwar beträgt die Wassermenge etwa das Zwanzigfache, bezogen auf die eingesetzte Carbonsäure. Die Abtrennung der ausgefällten Metallseife kann z.B. durch Filtrieren oder Zentrifugieren erfolgen. Der Anteil des ebenfalls gebildeten Alkalisalzes wird durch Auswaschen unterhalb 1% gebracht, anschliessend wird die Metallseife getrocknet und gemahlen. Das Endprodukt fällt in sehr leichter, voluminöser und, was besonders nachteilig anzusehen ist, in stark staubender Form an. Nachteilig sind an diesem Verfahren weiterhin die aufwendigen und zahlreichen Verfahrensschritte.

Es ist weiterhin ein Verfahren bekannt, bei dem die Umsetzung der Carbonsäure mit dem entsprechenden Metalloxid oder -hydroxid oder -carbonat in der Schmelze erfolgt. Dabei wird das Metalloxid oder -hydroxid oder -carbonat oberhalb des Schmelzpunktes des zu erwartenden Endproduktes der geschmolzenen Carbonsäure zugesetzt und sich bildendes Reaktions- und Hydratwasser durch Verdampfen entfernt. Die wasserfreie Schmelze wird anschliessend über Kühltürme, Schuppenwalzen oder Kühlbäder zum Erstarren gebracht und gemahlen. Nachteilig an diesem Verfahren ist, dass dabei ebenfalls staubende und relativ dunkelgefärbte Endprodukte erhalten werden. Der Einsatz dieser Produkte zu einer Weiterverarbeitung wird weiter, vor allem durch sein grobes Korn und der daraus resultierenden schlechten Verteilung, stark eingeschränkt.

In der DE-AS 1 068 238 wird ferner ein Verfahren zur Herstellung von Bleisalzen von Fettsäuren beschrieben. Dabei erfolgt die Umsetzung der Fettsäure in feinteiliger oder feinverteilter Form mit Bleioxid in wässriger Suspension bei Temperaturen knapp unterhalb der Schmelztemperatur der Säure. Die gebildete, unlösliche Bleiseife wird abgesaugt und anschliessend getrocknet. Nachteilig an diesem Verfahren ist wiederum die zur Erzielung der erforderlichen Feinteiligkeit der Fettsäure in wässriger Suspension notwendige grosse Wassermenge, die gemäss den angeführten Beispielen etwa das Sechs- bis Dreizehnfache, bezogen auf die eingesetzte Carbonsäure, beträgt. Auch bei diesem Verfahren wird kein direkt als Granulat anfallendes Endprodukt erhalten.

Weiterhin ist aus der DE-A 2 361 607 ein Verfahren der eingangs genannten Art bekannt. Bei diesem Verfahren wird das Metalloxid in der geschmolzenen Fettsäure dispergiert und dann das Wasser zugesetzt. Nach der Einleitung der Reaktion wird die Erwärmung des Gemisches bei 100°C fortgesetzt, und die Mischung wird zur Reaktion stehengelassen. Ein Trocknen des Reaktionsproduktes ist entweder nicht vorgesehen, oder es erfolgt eine Trocknung an der Luft oder beim Zermahlen. Bei diesem Verfahren werden die Reaktionsprodukte zermahlen, d.h. es werden keine direkt als Granulate anfallende Metallseifen erhalten.

Der vorliegenden Erfindung liegt die Aufgabe zugrunde, ein verbessertes Verfahren zum Herstellen von Metallseifen oder Metallseifenmischungen zu schaffen, welches die Nachteile der bekannten Verfahren nicht aufweist und in einfacher und kostensparender Weise, ohne einen zusätzlichen Arbeitsgang zu staubarmen, direkt als Granulate anfallenden weissen Metallseifen oder Metallseifenmischungen führt.

Erfindungsgemäss ist diese Aufgabe durch ein Verfahren der eingangs genannten Art dadurch gelöst, dass man zur Herstellung von direkt in Granulatform anfallenden weissen Metallseifen oder Metallseifengemischen die Ausgangskomponenten im Druckreaktor vermischt, das Gemisch auf die für das Anspringen der Umsetzung nötigen Temperatur erhitzt, die exotherme Reaktion im geschlossenen Druckreaktor unter Rühren und ohne weitere äussere Wärmezufuhr ablaufen lässt und nach dem Entspannen vom aufgebauten Überdruck das Wasser unter erhöhter Rührgeschwindigkeit und unter vermindertem Druck abzieht.

Die Vorteile des erfindungsgemässen Verfahrens gegenüber den bekannten Verfahren werden im folgenden dargelegt:

Als besonders nachteilig bei den bekannten Verfahren ist anzusehen, dass die Endprodukte in stark staubender, teilweise dunkelgefärbter Form anfallen.

Überraschenderweise liefert das erfindungsgemässe Verfahren direkt Endprodukte in granulierter Form. Dadurch zeigen diese Produkte keine Staubentwicklung und weisen bessere Eigenschaften auf wie durch doppelte Umsetzung in wässriger Phase oder direkte Umsetzung in der Schmelze hergestellte Metallseifen. Die Korngrössenverteilung der in Granulatform anfallenden Fertigprodukte nach dem erfindungsgemässen Verfahren ist ausserordentlich günstig, da von der Gesamtmenge etwas 80% auf die gewünschte Kornfraktion entfallen. Beispielsweise kann erfindungsgemäss ein Produkt hergestellt werden, bei dem etwa 80% der Metallseife in einer Teilchengrösse von 0,2 bis 2 mm anfallen.

Das erfindungsgemässe Verfahren zeichnet sich gegenüber den bekannten Verfahren weiterhin dadurch aus, dass es sowohl bei der Umsetzung, als auch bei der anschliessenden Trocknung energieeinsparend und somit wirtschaftlicher ist als die bekannten Verfahren. Einmal dadurch, dass bisher für die Umsetzung die zwanzigfache Menge Wasser, bezogen auf die eingesetzte Carbonsäure, mit aufgeheizt werden musste, zum anderen dadurch, dass beim Trocknen der Metallseife beim erfindungsgemässen Verfahren keine weitere Energiezufuhr benötigt wird, da die im Reaktorinhalt steckende Wärmemenge ausreicht, um bei Evakuierung des Reaktors das Wasser vollständig zu verdampfen.

Ferner erfolgt bei dem unter doppelter Umsetzung ablaufenden Verfahren die Trocknung der Metallseife mit konventionellen Trockenmethoden (z.B. über Band-, Strom-, Horden-, Trommel- oder Etagentrockner), und die dabei freiwerdenden feinsten Staubanteile belasten die Umwelt.

Die Herstellung der Metallseife oder Metallseifengemische nach dem erfindungsgemässen Verfahren wird von der Umsetzung bis hin zum fertigen Endprodukt in einem Behälter ausgeführt und ist somit einfacher und zeitsparender als die herkömmlichen Verfahren.

Eine zweckmässige Ausführungsform des erfindungsgemässen Verfahrens wird nachstehend beschrieben:

Eine feste aliphatische Carbonsäure mit 8 bis 22 Kohlenstoffatomen und/oder ein Gemisch dieser Carbonsäuren wird mit mindestens einem Metalloxid und/oder -hydroxid und/oder -carbonat unter Zusatz von 1 bis 5% Wasser, bezogen auf das Gesamtgewicht der Carbonsäure(n), in einem Druckreaktor vermischt und das Gemisch auf eine für das Anspringen der Umsetzung nötige Temperatur erhitzt. Die exotherme Reaktion läuft unter Rühren und ohne weitere äussere Wärmezufuhr bei einer Temperatur zwischen etwa 90 bis 110°C ab. Hierbei baut sich innerhalb von etwa 10 bis 15 Minuten ein Druck von etwa 1,1 bis 1,3 bar auf. Nach vollendeter Umsetzung wird vom aufgebauten Druck entspannt und das zugesetzte Wasser sowie entstandenes Reaktionswasser unter vermindertem Druck und erhöhter Rührgeschwindigkeit abgezogen. Dabei sinkt die Temperatur des Reaktionsproduktes relativ rasch auf etwa 50 bis 55°C, und man erhält Endprodukte in granulierter Form. Das erhaltene Granulat wird über ein Sieb fraktioniert und nicht erwünschte Grob- und Feinanteile, welche nur etwa 10% der Gesamtmenge betragen, wieder dem nächsten Reaktionsansatz zugeführt.

Bevorzugt werden beim erfindungsgemässen Verfahren 2 bis 4% Wasser, bezogen auf das Gesamtgewicht der Carbonsäure(n), zugesetzt, da bei diesen Mengen die günstigsten Ergebnisse erzielt werden.

Um die exotherme Reaktion beim erfindungsgemässen Verfahren starten zu können, muss erst Aktivierungsenergie in Form von Wärme zugeführt werden. Dabei hat es sich als besonders günstig gezeigt, wenn das Gemisch mindestens auf den Schmelzpunkt der eingesetzten Carbonsäure bzw. mindestens auf den Schmelzbereich des eingesetzten Carbonsäuregemisches, üblicherweise auf 50 bis 60°C, erhitzt wird.

Als feste aliphatische Carbonsäuren mit 8 bis 22 Kohlenstoffatomen werden vorzugsweise geradkettige, gegebenenfalls hydroxylgruppenhaltige Carbonsäuren eingesetzt.

Beim erfindungsgemässen Verfahren wird mindestens ein Oxid und/oder Hydroxid und/oder Carbonat aus der Gruppe Lithium, Natrium, Kalium, Magnesium, Calcium, Strontium, Barium, Zinn, Blei und Zink eingesetzt, da damit weisse Endprodukte erhalten werden.

Man verwendet einen geringen Überschuss von etwa 2% über die zur Säure äquivalente Menge mindestens eines Metalloxids und/oder -hydroxids und/oder -carbonats, um zu verhindern, dass nach der Umsetzung freie, nicht erwünschte Säuregruppen vorliegen.

Nach den bekannten Verfahren mussten die Metallseifen, wollte man sie in granulierter Form erhalten, in einem weiteren nachgeschalteten Arbeitsgang in schnell laufenden Mischern oder Verformungsaggregaten unter Zusatz von Granulierhilfsmitteln bei Temperaturen über dem Schmelzpunkt dieser Hilfsmittel granuliert, anschliessend abgekühlt und gesiebt werden. Nach dem erfindungsgemässen Verfahren fallen direkt weiche, rieselfähige und gut dosierbare Granulate an. Falls gewünscht, kann man die so erhaltenen Metallseifengranulate in üblicher Weise in demselben Reaktor nach Abziehen des Wassers, z.B. mit bekannten, sonst als Granulierhilfsmittel verwendeten Gleitmitteln, wie Paraffine oder Fette, überziehen.

Die Beispiele erläutern die Erfindung

Beispiel 1
Herstellung einer Calciumseife

25 kg eines technischen Carbonsäuregemisches der Kettenlänge $C_{14}$ bis $C_{20}$ (2% $C_{20}$, 64% $C_{18}$, 2% $C_{17}$, 28% $C_{15}$, 4% $C_{14}$) und einem mittleren Molekulargewicht von 273 werden mit 0,5 l Wasser und 3,5 kg Kalkhydrat in einem mit einem Rührer versehenen, 130 l fassenden Druckreaktor gegeben. Das Gemisch wird auf 50 bis 60°C aufgeheizt. Die Reaktion läuft dann ohne weitere Wärmezufuhr exotherm ab. Die Umsetzung wird unter Rühren durchgeführt, wobei der Druck in dem geschlossenen Reaktor auf 1,1 bis 1,3 bar ansteigt. Wenn der Druck bzw. die Temperatur wieder abzufallen beginnt, ist die Umsetzung vollständig. Der Reaktor wird dann entspannt und das enthaltene Wasser unter Rühren und vermindertem Druck abgezogen. Als Reaktionsprodukt wird direkt eine nichtstaubende Calciumseife in granulierter, rieselfähiger Form mit engem Korngrössenbereich erhalten.

Die nachstehenden Beispiele wurden gemäss Beispiel 1 durchgeführt.

**Beispiel 2**
**Herstellung einer Bleiseife**

25 kg desselben Carbonsäuregemisches wie in Beispiel 1 werden mit 0,5 l Wasser und 10,6 kg Bleiglätte umgesetzt.

**Beispiel 3**
**Herstellung einer Zinkseife**

25 kg desselben Carbonsäuregemisches wie in Beispiel 1 werden mit 0,5 l Wasser und 3,75 kg Zinkoxid umgesetzt.

**Beispiel 4**
**Herstellung einer Natriumseife**

30 kg desselben Carbonsäuregemisches wie in Beispiel 1 werden mit 0,5 l Wasser und 4,4 kg Natriumhydroxid (100%ig) umgesetzt.

**Beispiel 5**
**Herstellung einer Bariumseife**

30 kg desselben Carbonsäuregemisches wie in Beispiel 1 werden mit 0,5 l Wasser und 9,5 kg Bariumhydroxid umgesetzt.

Auch in den Beispielen 2 bis 5 wurden nichtstaubende, direkt in granulierter und rieselfähiger Form anfallende Metallseifen erhalten.

**Patentansprüche**

1. Verfahren zur Herstellung von Metallseifen oder Metallseifengemischen durch Umsetzen mindestens einer festen aliphatischen Carbonsäure mit 8 bis 22 Kohlenstoffatomen mit mindestens einem Metalloxid und/oder -hydroxid und/oder -carbonat aus der Gruppe Lithium, Natrium, Kalium, Magnesium, Calcium, Strontium, Barium, Zinn, Blei und Zink in mindestens einer zur Carbonsäure äquivalenten Menge bis zu einem 2%igen Überschuss in Gegenwart von 1 bis 5% Wasser, bezogen auf das Gesamtgewicht der Carbonsäure(n), bei erhöhter Temperatur, wobei man das Gemisch auf eine für das Anspringen der Umsetzung nötige Temperatur erhitzt, die exotherme Reaktion in einem geschlossenen Druckreaktor ablaufen lässt, anschliessend vom aufgebauten Überdruck entspannt und dann das im Reaktionsgemisch enthaltene Wasser entfernt, dadurch gekennzeichnet, dass man zur Herstellung von direkt in Granulatform anfallenden weissen Metallseifen oder Metallseifengemischen die Ausgangskomponenten im Druckreaktor vermischt, das Gemisch auf die für das Anspringen der Umsetzung nötige Temperatur erhitzt, die exotherme Reaktion im geschlossenen Druckreaktor unter Rühren und ohne weitere äussere Wärmezufuhr ablaufen lässt und nach dem Entspannen vom aufgebauten Überdruck das Wasser unter erhöhter Rührgeschwindigkeit und unter vermindertem Druck abzieht.

2. Verfahren nach Anspruch 1, dadurch gekennzeichnet, dass man 2 bis 4% Wasser, bezogen auf das Gesamtgewicht der Carbonsäure(n), einsetzt.

3. Verfahren nach einem der Ansprüche 1 oder 2, dadurch gekennzeichnet, dass man für das Anspringen der Umsetzung das Gemisch mindestens auf den Schmelzpunkt der eingesetzten Carbonsäure bzw. mindestens auf den Schmelzbereich des eingesetzten Carbonsäuregemisches erhitzt.

4. Verfahren nach einem der Ansprüche 1 bis 3, dadurch gekennzeichnet, dass man als aliphatische Carbonsäure(n) mit 8 bis 22 Kohlenstoffatomen geradkettige, gegebenenfalls hydroxylgruppenhaltige Carbonsäure(n) einsetzt.

**Claims**

1. A method for the manufacture of metallic soaps or metallic soap mixtures by reaction of at least one solid aliphatic carboxylic acid having 8 to 22 carbon atoms with at least one metallic oxide and/or hydroxide and/or carbonate of the group comprising lithium, sodium, potassium, magnesium, calcium, strontium, barium, tin, lead and zinc, in at least a quantity equivalent to the carboxylic acid, such quantity being up to a 2% excess, in the presence of 1 to 5% water based on the total weight of the carboxylic acid or acids, at elevated temperature, the mixture being heated to a temperature required for initiation of the reaction, the exothermic reaction is carried out in a closed pressure reactor, the excess pressure which has built up is released, and then the water contained in the reaction mixture is removed, characterised in that for the manufacture of white metallic soaps or metallic soap mixtures occurring directly in granulate form the feed ingredients are mixed in the pressure reactor, the mixture is heated to the temperature required for initiation of the reaction, the exothermic reaction is carried out in the closed pressure reactor with agitation and without further external heat supply and after the excess pressure that has built up has been released the water is removed at increased speed of agitation and at reduced pressure.

2. A method according to claim 1, characterised in that 2 to 4% of water is used based on the total weight of the carboxylic acid or acids.

3. A method according to claim 1 or 2, characterised in that to initiate the reaction the mixture is heated at least to the melting point of the carboxylic acid used or at least to the melting range of the carboxylic acid mixture used.

4. A method according to any one of claims 1 to 3, characterised in that the aliphatic carboxylic acid or acids having 8 to 22 carbon atoms used are straight-chain carboxylic acid(s), which may contain hydroxyl groups if required.

**Revendications**

1. Procédé pour la préparation de savons métalliques ou de mélanges de savons metalliques, par réaction d'au moins un acide carboxylique aliphatique solide ayant de 8 à 22 atomes de carbone, sur au moins un oxyde et/ou hydroxyde et/ou carbonate métallique, le métal appartenant au groupe comprenant le lithium, le sodium, le potassium, le magnésium, le calcium, le strontium, le baryum, l'étain, le plomb et le zinc, en une quantité comprise entre au moins une quantité équivalente à celle de l'acide carboxylique et un excès de 2%, en présence de 1 à 5% d'eau, par rapport au poids total du ou des acides carboxyliques, à température élevée, procédé dans lequel on porte le mélange à une température nécessaire au déclenchement de la réaction, on laisse se dérouler la réaction exothermique dans un réacteur sous pression, fermé, puis on détend la surpression qui s'est constituée, et on élimine l'eau contenue dans le mélange réactionnel, caractérisé en ce que, pour préparer des savons métalliques ou mélanges de savons métalliques, blancs, obtenus directement sous forme de granulés, on mélange les constituants de départ dans le réacteur sous pression, on chauffe le mélange à la température nécessaire à l'amorçage de la réaction, on laisse se dérouler la réaction exothermique dans le réacteur sous pression, fermé, sous agitation et sans apport supplémentaire de chaleur extérieure, et, après la détente de la surpression qui s'est constituée, on soutire l'eau sous une vitesse d'agitation élevée et sous pression réduite.

2. Procédé selon la revendication 1, caractérisé en ce qu'on utilisé de 2 à 4% d'eau, par rapport au poids total du ou des acides carboxyliques.

3. Procédé selon l'une des revendications 1 ou 2, caractérisé en ce que, pour l'amorçage de la réaction, on chauffe le mélange au moins au point de fusion de l'acide carboxylique utilisé, ou au moins jusqu'à l'intervalle de fusion du mélange d'acides carboxyliques utilisés.

4. Procédé selon l'une des revendications 1 à 3, caractérisé en ce qu'on utilise comme acide ou acides carboxyliques aliphatiques ayant de 8 à 22 atomes de carbone un ou plusieurs acides carboxyliques à chaîne droite, ayant de 8 à 22 atomes de carbone, contenant éventuellement des groupes hydroxyle.